# EUROPEAN PATENT SPECIFICATION

(11) **EP 1 278 523 B1**
(45) Date of publication and mention of the grant of the patent: **07.07.2004**
(21) Application number: 01934116.3
(22) Date of filing: 03.05.2001
(51) Int. Cl.: A61K 31/565, A61K 31/57, A61P 35/00

(54) **COMBINATION OF PROGESTERONE AND MIFEPRISTONE FOR CANCER THERAPY**
KOMBINATION DER PROGESTERON UND MIFEPRISTONE FÜR KREBS-THERAPIE
COMBINAISON DE PROGESTERONE ET MIFEPRISTONE DANS LE TRAITEMENT DU CANCER

(30) Priority: 03.05.2000 GB 0010683
(43) Date of publication of application: 29.01.2003
(73) Proprietor: The University of Bristol, Clifton, Bristol BS8 1TH (GB)
(72) Inventor: GASTON, Kevin, Bristol BS8 1TD (GB)
(74) Representative: Dean, John Paul
(86) International application number: PCT/GB2001/001941
(87) International publication number: WO 2001/082910

(56) References cited:
- WO-A-93/21926
- DE-A- 4 330 234
- YUAN, FANG (1) ET AL: "Altered growth and viral gene expression in human papillomavirus type 16-containing cancer cell lines treated with progesterone." CANCER INVESTIGATION, (1999) VOL. 17, NO. 1, PP. 19-29., XP001058156
- BARTHOLOMEW J S ET AL: "Integration of high-risk human papillomavirus DNA is linked to the down-regulation of class I human leukocyte antigens by steroid hormones in cervical tumor cells." CANCER RESEARCH, (1997 MAR 1) 57 (5) 937-42., XP001009724
- MICHELIN, DAVID ET AL: "Regulation of human papillomavirus type 18 in vivo: Effects of estrogen and progesterone in transgenic mice." GYNECOLOGIC ONCOLOGY, (1997) VOL. 66, NO. 2, PP. 202-208., XP001058118
- MITTAL, RAKESH ET AL: "Human papillomavirus type 16 expression in cervical keratinocytes: Role of progesterone and glucocorticoid hormones." OBSTETRICS & GYNECOLOGY, (1993) VOL. 81, NO. 1, PP. 5-12., XP001058158

## Description

### Field of the Invention

The present invention relates to a method of treatment of cervical cancer, pre-cancerous cervical lesions, vulva intraepithelial neoplasia (VIN), and cancer of the vulva.

### Background

Several types of human papillomavirus (HPV) are associated with cervical cancer (reviewed by van Ranst, *M. et al.,* (1996) *Papillomavirus Reviews: Current Research on Papillomaviruses*). HPV DNA sequences are found in malignant cervical lesions; HPV 16 is the most common genotype and accounts for around half of all cases of this disease (Bosch, F. X. *et al.,* (1995) *Journal of the National Cancer Institute,* **87,** 796-802); Walboomers, J. M. M. *et al.,* (1999) *The Journal of Pathology,* **189,** 12-19). HPV 16 DNA is also found in a high proportion of cases of vulval intraepithelial neoplasia (VIN) and cancers of the vulva. However, cancers do not produce viral particles and do not represent the typical outcome of an HPV 16 infection. In HPV-infected cells, the HPV genome exists as an extra-chromosomal DNA circle. In HPV-transformed cells from cervical tumours, viral DNA is often integrated into the host genome (Dürst, M. *et al.,* (1985) *Journal of General Virology,* **66,** 1515-1522). Integration frequently occurs within the viral E2 gene and results in loss of the E2 gene product (Baker, C. C. *et al.,* (1987) *Journal of Virology,* **61,** 962-971). These observations suggest that the absence of the E2 protein is a major factor in the development of cervical cancer (Schneider-Maunoury, S. *et al.,* (1987) *Journal of Virology,* **61,** 3295-3298).

The HPV E2 proteins are required for viral replication and are thought to play a role in the regulation of HPV gene expression (reviewed by Thierry, F. (1996) *Papillomavirus reviews: current research on papillomaviruses).* The E2 proteins have dramatic effects on cell survival. The HPV 16 E2 protein can induce apoptotic cell death in a variety of both HPV-transformed, and non-HPV-transformed, cell lines (Sanchez-Perez, A. M. *et al.,* (1997) *Journal of General Virology* **78,** 3009-3018; Webster, K. *et al.,* (2000) *Journal of Biological Chemistry,* **275,** 87-94). The E2 proteins from HPV 18 and HPV 33 have been shown to induce apoptosis in HeLa cells (an HPV 18-transformed cell line), and in normal human foreskin keratinocytes, respectively (Desaintes, C. *et al.,* (1997) *EMBO Journal* **16,** 504-514; Frattini, M. G. *et al.,* (1997) *EMBO Journal,* **16,** 318-331). Following HPV 16 infection the E2 protein could generate a pro-apoptotic signal within the infected cell.

The HPV 16 E6 protein can block E2-induced apoptosis (Webster, K. *et al.,* (2000) *Journal of Biological Chemistry,* **275,** 87-94). E6 binds to the p53 tumour suppressor protein and targets this protein for degradation (Schneffer, M. *et al.,* (1990) *Cell,* **63,** 1129-1136; Wemess, B. A. *et al.,* (1990) *Science,* **248,** 76-79; Lechner, M. S. *et al.,* (1992) *EMBO Journal,* **11**, 3045-3052; Hubbert, N. L. *et al.,* (1992) *Journal of Virology* **66**, 6237-6241). The HPV 16 E2 protein can interact physically with p53 and E2 is capable of inducing p53-dependent apoptosis (Massimi, P. *et al.,* (1999) *Oncogene,* **18,** 7748-7754; Webster, K. *et al.,* (2000) *Journal of Biological Chemistry,* **275,** 87-94). Thus, during viral infection any pro-apoptotic signal generated by E2 is probably counter-balanced by E6.

HPV 16 infection alone is insufficient to cause cervical cancer and several possible cofactors have been identified including the steroid hormones progesterone and estrogen. Progesterone can act as a cofactor in the transformation of baby rat kidney cells by HPV 16 and Ras (Pater, A. *et al.,* (1990) *American Journal of Obstetrics and Gynaecology,* **162,** 1099-1103). Progesterone has also been shown to increase HPV 16 and HPV 18 gene expression (Chen, Y. H*. et al.,* (1996) *EMBO Journal,* **16**, 504-514; Yuan, F. *et al.,* (1999) *Cancer Investigation,* **17,** 19-29; Mittal, R. *et al.,* (1993) *Obstetrics and Gynaecology,* **81**, 5-12). Most cases of cervical cancer arise in the most estrogen-sensitive region of the cervix; the transformation zone (Autier, P. *et al.,* (1996) *British Journal of Cancer,* **74**, 488-90 and references therein). Estrogen-containing oral contraceptives have been reported to double the risk of cervical cancer (Brisson, J. *et al.,* (1994) *American Journal of Epidemiology,* **140,** 700-710). Estrogens can increase HPV gene expression and increase the proliferation of cervical cancer cells *in vivo* (Mitrani-Rosenbaum, S. *et al.,* (1989) *Obstetrics and Gynaecology,* **81,** 5-12; Chen, Y. H. *et al.,* (1996) *Biochemical and Biophysical Research Communications,* **224,** 651-659; Bhattacharya, D. *et al.,* (1997) *British Journal of Cancer,* **75,** 554-558). Chronic estrogen exposure has been shown to induce cervical carcinogenesis in transgenic mice expressing the HPV 16 E6 and E7 genes (Arbeit, J. M. *et al.,* (1996) *Proceedings of the National Academy of Sciences of the USA,* **93**, 2930-2935).

The mechanism whereby estrogens act synergistically with E6 and/or E7 to induce cervical cancer is not known. One possibility is that high levels of DNA damage brought about by the estrogen metabolite 16α-hydroxyesterone result in the accumulation of mutations that lead to carcinogenesis (Aubourn, K. J. *et al.,* (1991) *International Journal of Cancer,* **49,** 867-869; Newfield, L. *et al.,* (1998) *Proceedings of the Society for Experimental Biology & Medicine,* **217,** 322-326). Estrogen is metabolised within the body to produce a family of related compounds, including 16α-hydroxyestrone. 16α-hydroxyesterone is estrogenic and has been shown to be tumourigenic in mice (Swaneck, G. E. & Fishman, J., (1988) *Proceedings of the National Academy of Sciences USA,* **85,** 7831-7835; Telang, N. T. *et al.,* (1992) *Journal of the National Cancer Institute,* **84,** 634-638). The transformation zone displays a high level of conversion of estradiol to 16α-hydroxyestrone and HPV-16 DNA increases this activity nearly 8-fold (Aubom, K. J. *et al.,* (1991) *supra*).

### Summary of the Invention

A first aspect of the invention provides the use of a progestogen, preferably progesterone or a progesterone analogue, and RU486 (Mifepristone) or an RU486 derivative in the preparation of a pharmaceutical composition for the treatment or amelioration of cervical cancer.

A second aspect of the invention provides the use of a progestogen, preferably progesterone or a progesterone analogue, and RU486 or an RU486 derivative in the preparation of a pharmaceutical composition for the treatment or amelioration of pre-cancerous lesions including cervical intraepithelial neoplasias grades I, II, and III and genital warts.

A third aspect of the invention provides the use of a progestogen, preferably progesterone or a progesterone analogue, and RU486 or an RU486 derivative and at least a portion of HPV E2 protein.

A fourth aspect of the invention provides a method of treatment of cervical cancer in a patient, comprising supplying to the patient a progestogen, preferably progesterone or a progesterone analogue and RU486 or an RU486 derivative, and at least a portion of HPV E2 protein. The E2 protein may be recombinantly produced or chemically synthesized.

A fifth aspect of the invention provides a method of treatment of vulva intraepithelial neoplasia (VIN) in a patient comprising supplying to the patient a progestogen, preferably progesterone or a progesterone analogue and RU486 or an RU486 derivative.

Progestogens include by way of example norethisterone, norgestrel, levonorgestrel, ethynodiol, desogestrel, gestodene, norgestimate and medroxyprogesterone. RU486 derivatives include RU633, RU848 and RU698.

Both the progestogen and RU486 (or derivative) are preferably supplied in an amount of 0.01 to 1.5mg per dose. A more preferable dose range is 0.375 mg to 0.5mg.

The steroid hormones may be bound to a steroid carrier protein or steroid carrier proteins.

The steroid carrier protein or steroid carrier proteins may be fused either via gene fusion or via chemical cross linking to at least a portion of HPV E2.

If a portion of HPV E2 is used, the portion should be any part of the protein in which the DNA-binding domain is absent.

A further aspect of the invention provides the use of a steroid hormone or steroid hormone analogue or analogues and at least a portion of HPV E2 protein in the preparation of a pharmaceutical composition for the treatment of cervical cancer.

A further aspect of the invention provides the use of a steroid hormone or steroid hormone analogue or analogues and at least a portion of HPV E2 protein in the preparation of a pharmaceutical composition for the treatment of pre-cancerous cervical lesions.

A further aspect of the invention provides a pharmaceutical composition comprising a steroid hormone or hormones or steroid hormone analogue or analogues and at least a portion of HPV E2 protein.

A further aspect of the invention provides a pharmaceutical composition comprising estrogen and/or estrogen analogue(s) or a progestogen, preferably progesterone and/or progesterone analogue(s), and at least a portion of HPV E2 protein.

The pharmaceutical composition of the invention may be provided in the form of a packaged liposome. Alternative formulations include nanoparticles, gels, creams, topical solutions and lotions, sprays, ointments, emulsions, suppositories/pessaries, injectable solutions and particles suitable for needle-free injection.

A further aspect of the invention provides a method of treatment of cervical cancer in a patient comprising supplying to a patient a steroid hormone or hormones or steroid hormone analogue or analogues and at least a portion of a human papillomavirus (HPV) E2 protein.

A further aspect of the invention provides a method of treatment of vulva intra-epithelial neoplasia (VIN) comprising supplying to a patient a steroid hormone or hormones or steroid hormone analogue or analogues and at least a portion of a human papillomavirus (HPV) E2 protein.

A further aspect of the invention provides a method of treatment of precancerous cervical lesions comprising supplying to a patient a steroid hormone or hormones or steroid hormone analogue or analogues and at least a portion of a human papillomavirus (HPV) E2 protein.

The steroid hormone may be estrogen, a progestogen, an androgen, or a mineralocorticoid. Progesterone or a progesterone analogue may also be supplied to the patient.

The steroid hormone(s) or analogue(s) and HPV E2 protein may be supplied to the patient in the form of packaged liposomes. This has the advantage of providing an efficient means of uptake of these compounds by the patient. Further, the liposome can be specifically targeted to selected cells, depending on the liposome coating composition.

The steroid hormone may be bound to a steroid carrier protein, such as sex hormone binding globulin (SHBG), human serum albumin or human normal globulin. These proteins protect the hormone from metabolism and inactivation.

The steroid hormone or analogue and E2 protein may be supplied together.

The steroid carrier protein or a fragment of the steroid carrier protein may be fused to E2 (or a fragment of E2).

### Brief Description of the Invention

Embodiments of the invention will now be described, by way of example only, and with reference to the accompanying figures 1(a) to 2(f), in which:
Figure 1(a) is a graph showing the percentage of apoptotic HeLa cells resulting from transfection with increasing concentrations of pWEB (an empty expression vector) and pWEB-E2 (an expression vector that expresses the HPV 16 E2 protein);
Figure 1(b) is a graph showing the percentage of apoptotic cells transfected with pWEB or pWEB-E2 resulting from incubation with various levels of progesterone;
Figure 1(c) is a graph showing the percentage of apoptotic HeLa cells transfected with pWEB or pWEB-E2 resulting from incubation with 2µM progesterone at various concentrations of RU486;
Figure 1(d) is a graph indicating the viability of HeLa cells grown in media containing various concentrations of progesterone, over time;
Figure 1(e) is a graph indicating the viability of HeLa cells grown in media containing various concentrations of RU486, over time;
Figure 1(f) is a graph indicating the viability of HeLa cells grown in media containing 2µM progesterone, followed by media containing progesterone and/or RU486 at various concentrations, over time;
Figure 2(a) is a graph showing the percentage of apoptotic cells in a population of HeLa cells transfected with pWEB-E2 or pWEB and incubated for 24 hours in media containing various concentrations of estrogen;
Figure 2(b) is a graph showing the percentage of apoptotic cells in a population of HeLa cells transfected with pWEB-E2 or pWEB and incubated for 24 hours in media containing various concentrations of 16α-hydroxyesterone;
Figure 2(c) is a graph showing the percentage of apoptotic cells in a population of HeLa cells transfected with pWEB-E2 or pWEB and incubated for 24 hours in media containing 400nM estrogen and various concentrations of 3-hydroxytamoxifen;
Figure 2(d) is a graph indicating the viability of HeLa cells grown in media containing various concentrations of estrogen, over time;
Figure 2(e) is a graph indicating the viability of HeLa cells grown in media containing various concentrations of 3-hydroxytamoxifen, over time; and
Figure 2(f) is a graph indicating the viability of HeLa cells grown in media containing varying concentrations of estrogen and 3-hydroxytamoxifen, over time.

### Embodiments of the Invention

1. HPV 16 E2 can induce cell death in a variety of cell types. E2-induced cell death shows all of the characteristic features of apoptosis: chromatin condensation, blebbing of the plasma membrane, and the appearance of cell fragments with sub-Go DNA content (Sanchez-Perez, A. M. *et al.,* (1997) *supra*; Desaintes, C. *et al.,* (1997) *supra*; Webster, K. *et al.,* (2000) *supra*).

In all of the following experiments, HeLa cells were maintained in Minimal Essential Medium (MEM: Sigma) supplemented with 10% Foetal Bovine Serum (FBS: Sigma) and penicillin (100,000 U/litre) and streptomycin (100mg/litre) at 37°C in 5% CO₂. In some cases, supplements (all obtained from Sigma, with the exception of 3-hydroxytamoxifen which was obtained from Research Biochemicals International (RBI)) were added to the media at the concentrations specifically indicated for each separate experiment.

Prior to transient transfection, cells were seeded at 3x10⁵ cells per well onto coverslips in 6 well plates and incubated overnight to obtain a sub-confluent culture. The liposome based reagent Tfx-20 (Promega) was used at a 3:1 liposome:DNA ratio in 1ml serum-free media per transfection, according to the manufacturer's instructions. After 30 hours, the coverslips were washed in phosphate buffered saline (PBS) and the cells fixed in 4% paraformaldehyde/PBS at 22°C for 30 minutes. Following further washes in PBS, the cells were stained with bisbenzimide (Hoechst No. 33258: Sigma) for 30 minutes. Finally the coverslips were washed in PBS and mounted onto microscope slides in 10µl MOWIOL (Calbiochem).

Where used, fluorescence microscopy was carried out using a Leica DM IRBE inverted epi-fluorescent microscope with FITC and DAPI filter sets and a 20x air objective (Leica).

Where MTT assays were carried out, this involved removal of the media, followed by incubation of the cells in 50µg/ml MTT (3-(4,5 dimethylthiazol 2-yl) 2,5 diphenyl tetrazolium bromide) for 2 hours at 37°C. The blue formazan crystals were extracted and dissolved in DMSO (dimethylsulfoxide), and the absorbance measured at 560nm wavelength using an ELISA plate reader (Mosmann, T. (1983) *Journal of Immunological Methods* **65,** 55-631983).

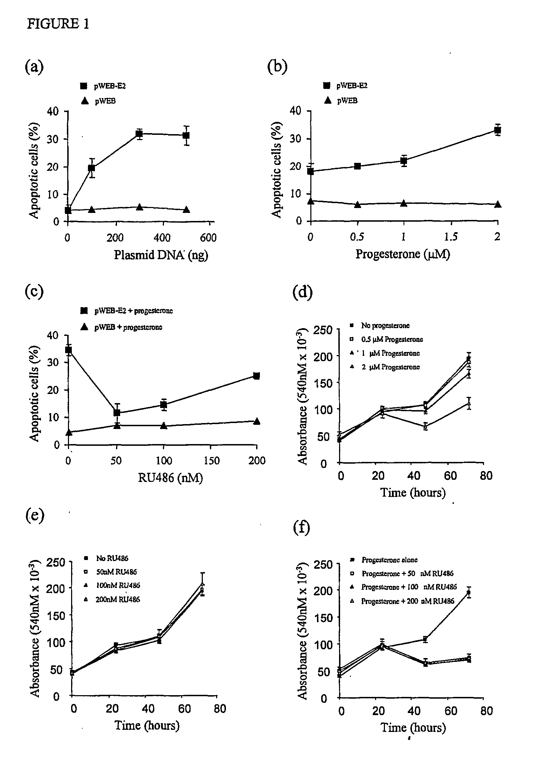

The plasmid pWEB-E2 expresses the HPV 16 E2 protein (Webster, K. *et al.,* (2000) *supra*). Increasing amounts of pWEB-E2 and the empty pWEB vector were transiently transfected into HeLa cells, an HPV 18-transformed cell cervical carcinoma cell line, along with a plasmid (pCMX-GFP3 - supplied by Dr Jeremy Tavaré of the Department of Biochemistry, University of Bristol, UK) that expresses the green fluorescent protein (GFP). After 30 hours the cells were fixed and then stained with bisbenzimide (Hoechst stain). Individual cells were scored as untransfected or transfected on the basis of GFP expression and then examined for chromatin condensation and membrane blebbing using Hoechst stain and GFP, respectively (Webster, K. *et al.,* (2000) *supra*). 100 transfected cells were counted and the experiment was repeated four times. The presence of pWEB-E2 results in an increase in the percentage of cells undergoing apoptosis compared to the cells transfected with the empty pWEB (Figure 1(a)).

To investigate any effects of steroid hormones on the induction of apoptosis by E2, HeLa cells were cultured in media containing 2µM progesterone for one week prior to transient transfection with pWEB-E2 or pWEB. Progesterone brings about a significant increase in the levels of E2-induced apoptosis (Figure 1(b). The level of apoptosis seen in cells transfected with the empty pWEB vector remains at around 7% suggesting that at these concentrations progesterone does not increase the background level of cell death.

Progesterone binds to the progesterone receptor and induces a conformational change that allows the hormone-receptor complex to bind to specific DNA sequences and regulate gene expression. RU486 (Mifepristone) binds with high affinity to the progesterone receptor and acts as a partial antagonist (reviewed by Cadepond, Y. H. *et al.,* (1997) *Annual Review of Medicine,* **48,** 129-156). To determine whether the progesterone receptor is required for the increase in E2-induced apoptosis, we cultured HeLa cells in 2µM progesterone for one week prior to transfection with the pWEB-E2 and pWEB plasmids. The transfected cells were then incubated in media containing 2µM progesterone and RU486. The cell population transfected with the E2 expression plasmid and incubated in 2µM progesterone showed high levels of apoptosis (Fig. 1(c)). In the presence of progesterone and 50nM RU486, the level of E2-induced apoptosis is reduced almost to the background levels seen in the untransfected population. We conclude that the progesterone receptor is required for progesterone to elicit its effect on E2-induced apoptosis.

At 100 nM and 200nM of RU486 the levels of apoptosis are raised compared to those at 50nM RU486. In the presence of progesterone and 200nM RU486 there is also a slight increase in the level of apoptosis seen in the untransfected cells. To examine the effects of RU486 and progesterone on cell proliferation, HeLa cells were cultured in media containing RU486 and/or progesterone and at 24 hour intervals the number of viable cells was determined using MTT assays (Mosmann, T. (1983) *supra*). The presence of progesterone results in a decrease in cell proliferation (Fig. 1(d)). The presence of RU486 has little effect on cell proliferation (Fig. 1(e)). Interestingly, continuous culture for 48 or 72 hours in the presence of both progesterone and RU486 severely reduces proliferation (Fig. 1(f)). These results suggest that the increased levels of cell death seen in the presence of progesterone and high concentrations of RU486, is probably due to toxicity of this combination of hormones rather than an effect of these hormones on E2-induced apoptosis.

These results indicate that cervical cancer and its precursor lesions may be treated using progesterone in combination with RU486.

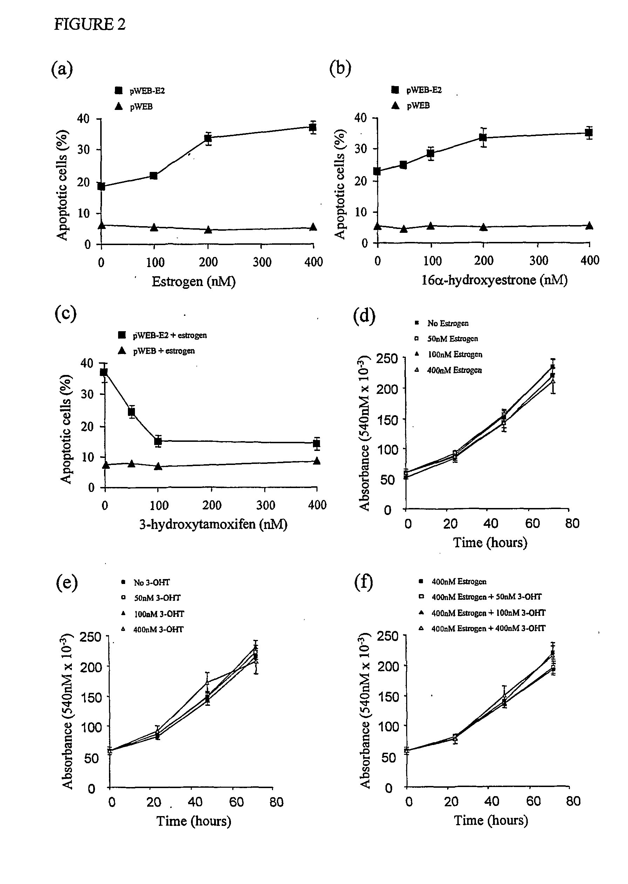

To investigate any effects of estrogen on E2-induced apoptosis, HeLa cells were transiently transfected with pWEB-E2 or pWEB and the cells incubated in media containing different concentrations of 17-β-estradiol, the primary active estrogen. Transfection with the E2 expressing plasmid results in a significant increase in the level of apoptosis when compared to cells transfected with the empty vector (Fig. 2(a)). The presence of estrogen resulted in a significant increase in the level of E2-induced apoptosis. The level of apoptosis in the cells transfected with pWEB remains at around 5-7% in both the presence and absence of estrogen.

*In vivo,* the oxidation of 17-β-estradiol to esterone is followed by hydroxylation to give 2-hydroxestrone or 16α-hydroxyesterone 2-hydroxesterone is a weak anti-estrogen. 16α-hydroxyestrone is highly estrogenic. To confirm and extend the results described above, studies were carried out to determine whether 16α-hydroxyestrone could mimic the effects of estrogen on E2-induced cell death. The addition of 16α-hydroxyestrone increases the level of E2-induced apoptosis (Fig. 2(b)).

Estrogens bind to estrogen receptors and the hormone-receptor complexes regulate the expression of target genes. The estrogen receptor antagonist 3-hydroxytamoxifen (3-OHT) binds to estrogen receptors and blocks their activity. The E2 expression plasmid was transfected into HeLa cells and the cells incubated in media containing estrogen and increasing concentrations of 3-OHT (Fig. 2(c)). Cells transfected with pWEB-E2 and incubated in the presence of 400nM estrogen, showed high levels of apoptosis. The percentage of apoptotic cells was reduced from around 30% in the presence of E2 and estrogen, to around 10% in the presence of E2, estrogen, and 400nM 3-OHT (Fig. 2(c)). The percentage of apoptotic cells in the pWEB-transfected population remained around 5% in the presence of estrogen and 3-OHT.

MTT assays were carried out to determine whether the concentrations of estrogen and 3-OHT used in these experiments have any effects on cell proliferation. Continuous culture for 72 hours in the presence of 400nM estrogen has no effect on the proliferation of HeLa cells (Fig. 2(d)). Similarly, MTT assays show that 3-OHT and the combination of 3-OHT and estrogen have no effect on the proliferation of these cells (Fig. 2(e) and 2(f), respectively).

These results indicate that cervical cancer and its precursor lesions may be treated using estrogen and/or progesterone in combination with E2.

### a) Formulations

### i. Progestogens and RU486

Formulations of progestogens and RU486 (or analogues) will usually contain a sufficient amount of the active compounds to provide from 0.375 to 0.5mg of each compound per dose.

Cream formulations comprise a hydrophobic phase, a hydrophilic phase and one or more surfactants. An example formulation comprises polyethylene glycol, glycol stearate, polyethylene glycol glycerides, mineral oil, benzoic acid, disodium EDTA, butylated hydroxyanisole and purified water.

Ointment formulations comprise a hydrophobic base. An example formulation comprises white soft paraffin, lanolin and liquid paraffin. Another example formulation contains simple ointment BP (or USP).

Gel formulations may be hydrophobic or hydrophilic. An example formulation contains hydroxypropylcellulose, butylated hydroxytoluene, ethanol and water. Another example contains a carbomer, propylene glycol, ethanol, laureth 4, diisopropanolamine and water. Hydrophobic gels may contain white soft paraffin and liquid paraffin. Alternative gelling agents include cellulosic starch and collagen.

An example topical solution contains isopropyl alcohol, propylene glycol and water. An example topical lotion contains polyol fatty acid ester, propylene glycol, ethanol, sodium hydroxide, disodium EDTA and water. An example emulsion formulation contains cetomacrogol 1000, cetostearyl alcohol, white soft paraffin, hard paraffin, propylene glycol, water and preservatives (such as parabens) and a buffer system (such as citric acid-citrate). Fatty acids such as stearic, palmitic or oleic acids may also be used to prepare emulsions.

Suppositories may be prepared using a theobroma oil or a witepsol base. A hydrophilic, e.g gelatinous, base may also be used. An example spray formulation is a pressurised aerosol-type formulation comprising liquid paraffin, coconut oil and hydrocarbon propellant. An alternative propellant is a hydrofluorocarbon. A non-aerosol 'pump' spray may also be prepared.

Injectable solutions comprise water, sodium chloride and a phosphate buffer, along with appropriate stabilising additives as described in the BP and USP. Nanoparticles may be polymeric or may be inorganic, for example, titanium dioxide nanoparticles.

### ii. Steroid Hormone and HPV E2 Protein

The compositions comprising steroid hormones and/or their analogues and at least a portion of HPV E2 protein may be formulated as described above. However, more preferred formulations include polymeric nanoparticles and packaged liposomes. Polymeric nanoparticles containing the hormones and the protein may be prepared using polyesters (e.g polylactide, polyglycolide or polylactide co-glycolide systems), polyanhydrides or polyorthoesters. The nanoparticles may be formed using well-known methods, such as emulsification followed by solvent evaporation, or coacervation.

Liposomes are prepared using naturally occurring or synthetic lipids. Naturally occurring phospholipids are preferred, including those based on phosphatidylethanolamine and phosphatidylcholine as would be present in lecithin. The liposomes may be formed by the shaking of a hydrophilic solution of the active compounds in the presence of a lipid film, optionally followed by sonication. The steroids may be incorporated into the lipid phase. The liposomes may also be formed by a reverse phase evaporation procedure.

Both nanoparticles and liposomes may include targeting moieties and/or hydrophilic polymer chains on their surfaces. These may be covalently bound to the lipid or polymer components prior to formulation, or may be attached afterwards. The hydrophilic polymer may comprise polyethylene glycol and acts to prevent the formulation being cleared by the immune system.

### b) Methods of Treatment

Active ingredients may be supplied sequentially, but are preferably supplied together.

## Claims

1. Use of a progestogen, and RU486 (Mifepristone) or an RU486 derivative in the preparation of a pharmaceutical composition for the treatment or amelioration of cervical cancer.

2. Use of a progestogen, and RU486 (Mifepristone) or an RU486 derivative in the preparation of a pharmaceutical composition for the treatment or amelioration of pre-cancerous lesions.

3. Use of a progestogen, and RU486 (Mifepristone) or an RU486 derivative according to claim 2, in which the pre-cancerous lesions are pre-cancerous vulva intraepithelial neoplasia or cervical lesions.

4. Use of a progestogen, and RU486 (Mifepristone) or an RU486 derivative and at least a portion of HPV E2 protein in the preparation of a pharmaceutical composition for the treatment of cervical cancer or pre-cancerous cervical lesions.

5. Use of a progestogen and RU486 (Mifepristone) or an RU486 derivative in the preparation of a pharmaceutical composition for the treatment of VIN.

6. Use of a progestogen according to any preceding claim in which the progestogen is progesterone or a progesterone analogue.

## Patentansprüche

1. Verwendung eines Progestogens und RU486 (Mifepristone) oder eines RU486-Derivats bei der Herstellung einer pharmazeutischen Zusammensetzung zur Behandlung oder Linderung von Zervixkarzinom.

2. Verwendung eines Progestogens und RU486 (Mifepristone) oder eines RU486-Derivats bei der Herstellung einer pharmazeutischen Zusammensetzung zur Behandlung oder Linderung von prekanzerösen Lesionen.

3. Verwendung eines Progestogens und RU486 (Mifepristone) oder eines RU486-Derivats nach Anspruch 2, wobei die prekanzerösen Lesionen prekanzeröse intraepitheliale Neoplasie der Vulva oder Zervixlesionen sind.

4. Verwendung eines Progestogens und RU486 (Mifepristone) oder eines RU486-Derivats und mindestens eines Teils des HPV E2-Proteins bei der Herstellung einer pharmazeutischen Zusammensetzung zur Behandlung von Zervixkarzinom oder prekanzerösen Zervixlesionen.

5. Verwendung eines Progestogens und RU486 (Mifepristone) oder eines RU486-Derivats bei der Herstellung einer pharmazeutischen Zusammensetzung zur Behandlung von VIN.

6. Verwendung eines Progestogens nach einem der vorhergehenden Ansprüche, wobei das Progestogen Progesteron oder ein Progesteron-Analog ist.

## Revendications

1. Utilisation d'une progestogène et du RU486 (Mifépristone) ou d'un dérivé du RU486 dans la préparation d'une composition pharmaceutique pour le traitement ou l'amélioration du cancer cervical.

2. Utilisation d'une progestogène et du RU486 (Mifépristone) ou d'un dérivé du RU486 dans la préparation d'une composition pharmaceutique pour le traitement ou l'amélioration de lésions pré-cancéreuses.

3. Utilisation d'une progestogène et du RU486 (Mifépristone) ou d'un dérivé du RU486 selon la revendication 2, pour laquelle les lésions pré-cancéreuses sont des néoplasies intraépithéliales pré-cancéreuses de la vulve ou des lésions cervicales.

4. Utilisation d'une progestogène et du RU486 (Mifépristone) ou d'un dérivé du RU486 et d'au moins une partie de la protéine PVH E2 dans la préparation d'une composition pharmaceutique pour le traitement du cancer cervical ou de lésions cervicales pré-cancéreuses.

5. Utilisation d'une progestogène et du RU486 (Mifépristone) ou d'un dérivé du RU486 dans la préparation d'une composition pharmaceutique pour le traitement des NIV.

6. Utilisation d'une progestogène selon l'une quelconque des revendications précédentes pour laquelle la progestogène est la progestérone ou un analogue de la progestérone.
